# FASCICULE DE BREVET EUROPEEN

(11) **EP 3 102 671 B1**
(45) Date de publication et mention de la délivrance du brevet: **01.07.2020**
(21) Numéro de dépôt: 14724120.2
(22) Date de dépôt: 10.04.2014
(51) Int. Cl.: C12N 1/16, C12N 1/18

(54) **LEVURE BIOLOGIQUE, PROCÉDÉ D'OBTENTION ET UTILISATIONS**
BIOLOGISCHE HEFE, VERFAHREN ZUR HERSTELLUNG DAVON UND VERWENDUNGEN DAVON
BIOLOGICAL YEAST, METHOD FOR OBTAINING SAME AND USES THEREOF

(30) Priorité: 06.02.2014 FR 1450911
(43) Date de publication de la demande: 14.12.2016
(73) Titulaire: Lesaffre et Compagnie, 75001 Paris (FR)
(72) Inventeur: BUGEON, Amélie, 63300 Thiers (FR); PETIT, Eric, F-59520 Marquette Lez Lille (FR)
(74) Mandataire: Plasseraud IP
(86) Numéro de dépôt international: PCT/FR2014/050869
(87) Numéro de publication internationale: WO 2015/118233

(56) Documents cités:
- EP-A1- 0 852 910
- BE-A- 421 525
- HAHN-HÄGERDAL BÄRBEL ET AL: "Role of cultivation media in the development of yeast strains for large scale industrial use", MICROBIAL CELL FACTORIES, BIOMED CENTRAL, LONDON, NL, vol. 4, no. 1, 10 novembre 2005 (2005-11-10), page 31, XP021007223, ISSN: 1475-2859, DOI: 10.1186/1475-2859-4-31
- CHU GYO-MOON ET AL: "Brewer's yeast efficiently degrades phytate phosphorus in a corn-soybean meal diet during soaking treatment", ANIMAL SCIENCE JOURNAL, vol. 80, no. 4, 2009, pages 433-437, XP002730587, ISSN: 1344-3941
- GARCIA-ESTEPA ROSA MA ET AL: "Phytic acid content in milled cereal products and breads", FOOD RESEARCH INTERNATIONAL, vol. 32, no. 3, 1999, pages 217-221, XP002730588, ISSN: 0963-9969

## Description

### Domaine technique de l'invention

La présente invention concerne un procédé de production de levure. Elle concerne en particulier un procédé de production de levure biologique comprenant l'utilisation de substrats d'origines biologiques en particulier un substrat biologique permettant de compléter les besoins nutritionnels de la levure en phosphore. Le procédé de la présente invention permet l'obtention de levure et extraits de levure biologiques conformes au REGLEMENT (CE) 834/2007 de l'Union Européenne.

### Arrière-plan technologique / problème à résoudre

BE 421525 décrit l'emploi de phytine ou d'acide phytique comme aliment pour la levure.

EP 0 852 910 décrit une composition azotée résultant de l'hydrolyse du gluten de blé et son procédé de fabrication.

Le document Bärbel Hahn-Hägerdal décrit le rôle des milieux de culture dans le développement de souches de levure pour des usages industriels.

Pour se multiplier la levure a besoin de sources de carbone et d'énergie, tél un mélange de sucres glucose, fructose et saccharose. Elle a aussi besoin d'azote, de phosphore, d'oxygène et d'autres oligoéléments dont entre autres le magnésium, le sodium, le potassium, le zinc, le cuivre ou encore le manganèse et facteurs de croissance dont la biotine, l'inositol, l'acide pantothénique, la thiamine, la pyridoxine, l'acide nicotinique voire l'acide para-amino benzoïque. Dans le cadre de la production classique de levures, on utilise en général des mélasses de canne à sucre ou de betterave comme source combinée de carbone et d'énergie. Ces mélasses apportent à la levure l'essentiel de ses besoins en carbone, en minéraux, en oligoéléments et en vitamines.

Le taux d'azote de la levure varie de 6 à 9% de la matière sèche de la levure soit de 37 à 56% de protéines. Or, l'apport azoté des mélasses est largement insuffisant. Par conséquent l'apport d'azote dans le milieu de culture se fait habituellement sous forme d'hydroxyde ou autres sels d'ammonium ou encore d'urée.

La mélasse manque de phosphore. La composition en phosphore de la levure exprimée en P₂O₅ est, de manière générale, le tiers de celle de l'azote, soit de 2 à 3% de la matière sèche. Le phosphore est, en général, ajouté sous forme d'acide phosphorique ou de ses sels.

La réglementation européenne impose des règles strictes pour la fabrication de levure biologique, qu'elle soit destinée à l'alimentation humaine ou à l'alimentation animale et quelle que soit l'application à laquelle la dite levure est destinée (panification, extraits de levures, vinification etc...).

Ainsi, seuls les substrats produits selon le mode biologique sont utilisables (REGLEMENT (CE) N° 834/2007). Toutefois et à titre dérogatoire (REGLEMENT (CE) N° 889/2008 après modification par le REGLEMENT (CE) N°1254/2008), 5% d'extrait ou autolysat de levure (EXL) non biologique calculé sur la base de la matière sèche des substrats biologiques sont autorisés tant que les opérateurs ne sont pas en mesure d'obtenir de l'extrait ou de l'autolysat de levure issu de la production biologique.

Cette dérogation d'utilisation d'EXL non biologique a été introduite par le législateur afin de compléter les apports en azote, phosphore, vitamines et minéraux absolument nécessaires à la production de levure biologique.

En effet, la fabrication industrielle de levure n'est envisageable que si les besoins nutritionnels de la levure sont parfaitement couverts.

Lorsque les substrats biologiques utilisés pour la fabrication de levure biologique sont les mélasses de canne et de betterave ainsi qu'une source de protéines isolée d'un produit agricole issu de l'agriculture biologique, ces substrats apportent en quantité suffisante le sucre, l'azote et une partie des minéraux et vitamines nécessaires à la croissance.

Par contre, le besoin en phosphore n'est pas satisfait. Dans ce cas, l'utilisation de l'autolysat de levure permet de le couvrir. Cependant, même en sélectionnant un autolysat particulièrement riche en phosphore, la limitation pondérale de son emploi ne couvre qu'imparfaitement les besoins d'une fabrication industrielle et de fait en limite la qualité et la régularité.

La fourniture d'un substrat riche en phosphore produit selon le mode biologique permettrait de résoudre le problème cité précédemment et de produire ainsi des levures et extraits de levures biologiques conformément aux Règlements de l'Union Européenne.

La demanderesse a trouvé que certains produits d'origine biologique riches en acide phytique sont, après solubilisation et hydrolyse, une source en phosphore assimilable efficacement par la levure.

### Résumé de l'invention

L'objet de l'invention est un procédé de culture de levure biologique comprenant l'utilisation des sources de carbone, d'azote et de phosphore d'origines biologiques, tel que défini à l'une quelconque des revendications 1 à 13.

La source de phosphore d'origine biologique selon l'invention est une solution purifiée riche en phosphore obtenue par un procédé comprenant au moins une étape de solubilisation et d'hydrolyse d'un substrat ou d'un mélange de substrats d'origine biologique riche(s) en acide phytique.

L'invention décrit une solution purifiée d'origine biologique riche en phosphore assimilable par la levure.

L'invention décrit également l'utilisation de la solution purifiée d'origine biologique riche en phosphore pour la production de levure biologique.

L'invention décrit encore une levure biologique conforme aux règlements de l'Union Européenne.

L'invention décrit aussi un extrait de levure biologique conforme aux règlements de l'Union Européenne.

### Description détaillée de l'invention

L'objet de l'invention est un procédé de production de levure biologique comprenant l'utilisation de substrats d'origines biologiques aptes à apporter tous les nutriments nécessaires à la croissance de la levure tel que défini à l'une quelconque des revendications 1 à 13.

Ces substrats sont, de manière préférentielle, de la mélasse comme source de sucres, une source de protéines biologiques hydrolysées comme source d'azote et au moins une solution purifiée riche en phosphore comme source de phosphore.

Le procédé de l'invention comprend en outre l'utilisation d'autres substances nécessaires à la croissance de la levure choisies parmi celles autorisées par la réglementation européenne telles que le carbonate de sodium, les acides lactique ou citrique et les huiles végétales.

Le procédé de l'invention met en œuvre de la mélasse d'origine biologique comme source de sucre. Selon une forme de l'invention, la mélasse utilisée est choisie parmi la mélasse de canne et la mélasse de betterave. Afin de mettre à profit leurs compositions différentes en termes de minéraux et vitamines, il est préférable selon l'invention d'utiliser conjointement les mélasses de canne et de betterave dans un rapport compris entre 50/50 et 80/20. Selon une forme préférée de l'invention le rapport mélasse de canne/mélasse de betterave est compris entre 65/35 et 75/25, et plus préférentiellement proche de 70/30.

Selon l'invention, la source biologique d'azote est une source de protéines biologiques hydrolysées, choisie parmi les protéines de riz, de pois, de pomme de terre, de blé, de soja, de luzerne, de spiruline et du gluten.

Selon une forme préférentielle de l'invention, la source biologique d'azote est le gluten hydrolysé.

L'hydrolyse de la source biologique d'azote peut être opérée par un cocktail enzymatique permettant d'avoir un taux de solubilisation de la matière sèche proche de 80% et un rendement en azote de 80 à 85%. Ce cocktail enzymatique comprend un mélange d'endoprotéases et d'exopeptidases, préférentiellement tout ou partie du mélange Neutrase, Alcalase, Cristalase et Flavourzyme. Ces enzymes, non OGM, sont autorisées pour la fabrication d'hydrolysats de protéines.

Selon l'invention, l'apport en phosphore est couvert par l'utilisation d'une solution, d'origine biologique, purifiée et riche en phosphore. Cette solution est obtenue par solubilisation et hydrolyse d'un substrat végétal d'origine biologique riche en acide phytique, le phosphore étant libéré sous forme de phosphate inorganique après solubilisation et hydrolyse de l'acide phytique.

Selon l'invention, par substrat biologique riche en acide phytique on entend un substrat végétal d'origine biologique comprenant de 2 à 18g de phosphore par KG de substrat dont de 60 à 80% sont sous la forme d'acide phytique.

Le substrat riche en acide phytique selon l'invention est choisi dans le groupe de végétaux listés dans le tableau I, suivant :

**Tableau I : Teneurs en phosphore total, ratio phosphore phytique sur phosphore total et activité phytasique de différentes matières premières (selon Sauvant 2002).**

| Nom | P (g/kg brut) moyenne (écart type) | P phytique / P total (%) | Activité phytasique (U/kg) | Groupe d'intérêt |
|---|---|---|---|---|
| Gluten de maïs | 8,9 (1,5) | 65 | 0 | 1 |
| Son de riz gras | 16,1 (2,1) | 85 | 120 | 1 |
| Graine de colza | 6,6 (0,9) | 70 | 0 | 1 |
| Tourteau de colza | 11,4 (0,9) | 60 | 10 | 1 |
| Tourteau de soja | 6,2 (0,5) | 60 | 20 | 1 |
| Tourteau de tournesol | 10,1 (1,4) | 85 | 0 | 1 |
| Remoulage demi-blanc de blé tendre | 8,7 (1,4) | 80 | 2590 | 2 |
| Son de blé | 9,9 (1,1) | 80 | 1770 | 2 |
| Seigle | 3 (0,3) | 65 | 5350 | 3 |
| Farine basse de blé tendre | 3,6 | 80 | 3080 | 3 |

Comme indiqué dans le tableau ci-dessus, certains substrats riches en acide phytique présentent une activité phytasique plus ou moins marquée. Le substrat d'origine biologique riche en acide phytique selon l'invention sera choisi soit selon :
- sa teneur importante en phosphore (groupe d'intérêt 1) ;
- sa teneur importante en phosphore et sa richesse en activité phytasique (groupe d'intérêt 2) ;
- sa richesse en activité phytasique (groupe d'intérêt 3).

Outre le phosphore, essentiellement présent sous la forme d'acide phytique, ces composés contiennent aussi des protéines voire de l'amidon, composés très utiles pour la croissance de la levure.

Par exemple, la composition moyenne du son de blé et du tourteau de soja est :
pour le son de blé :
   - teneur en matière sèche 87% sur produit tel que (TQ)
   - phosphore : environ 1% (TQ), soit environ 10g de phosphore par Kg de produit, sous forme d'acide phytique à 80%. Activité phytasique 1770 U/kg
   - protéines : 15 à 18% (TQ)
   - amidon : 20% (TQ)
pour le tourteau de soja:
   - teneur en matière sèche : 88 à 93% (TQ)
   - phosphore : 0,6% (TQ), (soit environ 6g de phosphore par Kg) sous forme d'acide phytique (60%) et de phospholipides. Activité phytasique 20 U/kg
   - protéines : 41% (TQ).

Il est nécessaire d'hydrolyser ces composés pour libérer les substances assimilables par la levure, car ni l'acide phytique ni les protéines et l'amidon ne sont assimilables en l'état par la levure.

L'acide phytique, de formule brute C₆H₁₈O₂₄P₆, est constitué d'un noyau inositol et de 6 groupements phosphates (InsP6). Sous l'action d'une phytase, l'acide phytique est hydrolysé sous forme de monophosphate inorganique et de myo-inositols phosphate de degré de phosphorylation inférieur (InsP5 à InsP1) et en myo-inositol libre dans certains cas comme décrit dans EP 1 910 531 B1.

La phytase employée pour cette hydrolyse peut être endogène à l'un des substrats riches employé dans le mélange à hydrolyser ou exogène dans le cas où le mélange de végétaux à hydrolyser serait déficient en activité phytasique endogène.

A noter que le son de blé ne nécessite pas l'emploi de phytase exogène. La solubilisation du phosphore du son de blé sans recours à une activité phytasique exogène est particulièrement intéressante car l'approvisionnement en phytase non issue d'OGM (obligation du règlement biologique) est difficile.

Le mélange de végétaux, tels ceux décrits dans le tableau I, peut permettre l'apport d'activité phytasique quand bien même le végétal utilisé ne pourrait être de prime abord qualifié de riche en phosphore (substrats du groupe d'intérêt 3 du tableau I).

La solubilisation et l'hydrolyse de l'acide phytique sont réalisées selon le mode comprenant au moins les étapes suivantes :
- le broyage du substrat biologique riche en acide phytique,
- la mise en suspension dans l'eau et chauffage de la suspension, et
- la désactivation enzymatique.

A titre indicatif, le broyage est réalisé à l'aide d'un broyeur à marteau équipé d'une grille à 800µm. Mais tout type de broyeur peut être utilisé.

La mise en suspension dans l'eau est opérée à raison de 100 à 250g et de préférence de 160 à 180g de produit broyé par Kg de suspension finale. La suspension est chauffée à une température comprise entre 40 et 50°C durant 5 à 20 heures.

Pour la désactivation enzymatique, la suspension est portée à une température de 90°C pendant une durée allant de 15 à 30 minutes, permettant en outre la pasteurisation du substrat.

Il est parfois nécessaire d'ajouter à la suspension une phytase exogène de manière à renforcer ou à compléter l'activité phytasique endogène. Dans ce cas, la phytase exogène est utilisée à raison de 15 à 25g par Kg de produit broyé.

A l'issue de ces traitements, les suspensions sont décantées et/ou clarifiées et/ou filtrées.

Afin de maximiser la récupération de la matière solubilisée, les boues de décantation, les boues de clarification centrifuge ou les gâteaux de filtration peuvent être lavés, les eaux de lavages sont alors rassemblées avec le surnageant initial. L'ensemble des solutés peut ensuite être concentré.

L'analyse des surnageants et eaux de lavages concentrés récoltés montre que 80% à 90% du phosphore contenu dans le substrat initial est solubilisé.

Selon un mode préféré de l'invention, le substrat riche en acide phytique est le son de blé.

Dans certains cas, la solubilisation du phosphore peut ensuite être complétée par les traitements plus classiques d'hydrolyse des protéines (mélange d'endo et d'exopeptidases) et de saccharification de l'amidon (utilisation d'un mélange d'amylase et d'amyloglucosidase).

L'invention décrit l'utilisation d'une solution purifiée riche en phosphore, telle que décrite précédemment, dans un procédé de production de levure, en particulier dans un procédé de production de levures biologiques comprenant l'utilisation de substrats d'origine biologique.

Le procédé de production de levure de l'invention est mis en œuvre selon les conditions de culture habituellement utilisées pour la production de levure traditionnelles, de même en ce qui concerne les conditions opératoires de récupération, séchage et de conditionnement des levures produites.

La présente invention permet en particulier le découplage des apports des ingrédients principaux nécessaires à la croissance de la levure que sont le sucre, la source d'azote, la source de phosphore et l'air. Ce découplage est recherché afin de maîtriser la composition finale de la levure fabriquée.

L'invention décrit également la levure biologique telle que produite par le procédé de l'invention.

L'invention décrit encore l'utilisation de la levure biologique de l'invention dans le domaine de la panification, de la production d'alcool, et plus généralement pour l'alimentation humaine et l'alimentation animale.

Les levures de l'invention sont particulièrement utiles pour la production d'extraits de levures biologiques.

L'invention décrit aussi un extrait de levure biologique obtenu à partir des levures biologiques de l'invention.

Les exemples suivants illustrent l'invention sans en limiter la portée.

### Exemples

### Exemple 1 : Solubilisation et hydrolyse du gluten

Il est connu qu'une hydrolyse de protéines végétales par une protéase purifiée (type papaïne ou alcalase) peut être améliorée par addition de levure en autolyse (EP0578572A, US201202888587).

Afin de démontrer l'efficacité du cocktail enzymatique proposé par l'invention, la demanderesse a réalisé les trois essais suivants :
- Recette 1 : gluten d'origine Blattman, levure biologique de brasserie mise en autolyse, papaïne
- Recette 2 : gluten d'origine Celnat, levure biologique de boulangerie mise en autolyse, papaïne (cristalase), neutrase et alcalase
- Recette 3 : gluten d'origine Celnat et/ou Blattman, Neutrase, Alcalase, Flavourzyme.

Dans un premier temps (recette 1) seule de la levure biologique de brasserie mise en autolyse a été utilisée en association avec de la papaïne (cristalase). Ensuite dans un deuxième temps, et afin de compenser la perte des activités protéolytiques des cellules de levures de brasserie, deux enzymes supplémentaires ont été employées (neutrase et alcalase) en association avec de la levure biologique de boulangerie mise en autolyse (recette 2).

Enfin, la papaïne a été remplacée par Flavourzyme (mélange d'endoprotéase et d'exopeptidase) et l'emploi de crème de levure biologique de boulangerie préalablement mise en autolyse a été abandonné.

Les bilans matières de ces 3 recettes d'hydrolyse sont rassemblés dans le tableau II.

**Tableau II : Résultats globaux des différentes recettes d'hydrolyse du gluten employées au cours des essais levure biologique.**

| **Recette** | **Recette 1** | **Recette 2** | **Recette 3** | | |
|---|---|---|---|---|---|
| Matières premières (en kgMS/T hydrolysat final non clarifié) | | | | | |
| Gluten | | | | | |
| - nature | Blattman | Celnat | Celnat | Celnat | Blattman |
| - teneur en MS | 93,9% | 93,4% | 93,6% | 94,1% | 92,6% |
| - concentration | 23 kgMS/T | 103 kgMS/T | 167 kgMS/T | 138 kgMS/T | 152 kgMS/T |
| - ratio MS | 50% | 90% | 100% | 100% | 100% |

| Levure | | | | | |
|---|---|---|---|---|---|
| - nature | Brasserie | Boulangère | | | |
| - concentration | 23 kgMS/T | 10 kgMS/T | | | |
| - ratio MS | 50% | 10% | | | |

| Enzymes (en kgMS/T MS à hydrolyser) | | | | | |
|---|---|---|---|---|---|
| Hydrolyse protéine | | | | | |
| - Papaïne | 3,34 kgMS/T | 21,43 kgMS/T | | | |
| - Neutrase | | 19,10 kgMS/T | 3,20 kgMS/T | 4,78 kgMS/T | 4,85 kgMS/T |
| - Alcalase | | 17,85 kgMS/T | 2,58 kgMS/T | 3,86 kgMS/T | 3,92 kgMS/T |
| - Flavourzyme | | | 0,11 kgMS/T | 0,16 kgMS/T | 0,16 kgMS/T |

| Solubilisation | | | | | |
|---|---|---|---|---|---|
| Matière sèche | | | | | |
| - teneur (kgMS/T | 43,0 kgMS/T | 99,0 kgMS/T | 153,3 kgMS/T | 128,2 | 135,5 |
| surnageant) | | | | kgMS/T | kgMS/T |
| - rendement (% | 81% | 73% | 80% | 83% | 79% |
| de MS engagée) | | | | | |

| Azote | | | | | |
|---|---|---|---|---|---|
| - rendement (% | 86% | 82% | 80% | 84% | 85% |
| de N engagé) | | | | | |

| Taux de boues après centrifugation labo 4000 G - 10 min (en kgMS/T hydrolysat non clarifié) | | | | | |
|---|---|---|---|---|---|
| - taux | 7 kgMS/T | 31 kgMS/T | 38 kgMS/T | 25 kgMS/T | 29 kgMS/T |
| - teneur en MS | 10% | 28% | 30% | 27% | 28% |
| boues | | | | | |

En termes de solubilisation de la matière sèche mise en œuvre, seule la recette 2 a présenté une moindre efficacité (73% contre 80-83%).

En termes de solubilisation de l'azote, il n'apparaît pas de différence nette entre les trois recettes testées. L'emploi de Flavourzyme semble donc globalement remplacer efficacement l'utilisation de crème de levure (brasserie ou biologique recyclée, préalablement mises en autolyse).

La recette 3 permet un rendement en azote proche de 85% ce qui est largement suffisant et un taux de dégradation des protéines de 39% assez satisfaisant, le taux de dégradation étant le rapport entre la teneur de l'hydrolysat en azote aminé (issu de l'hydrolyse) et la teneur de l'hydrolysat en azote total.

### Exemple 2 : Solubilisation et hydrolyse de son blé et de tourteau de soja

Les conditions opératoires des différents traitements appliqués ainsi que les résultats obtenus sont décrits ci-après :
- son de blé :
   ▪ broyage du son. A titre d'illustration, un broyeur à marteaux équipé d'une grille à 800µm a été utilisé lors des essais Pilote.
   ▪ mise en suspension dans l'eau à raison de 165g de son broyé par kg de suspension.
   ▪ chauffage 40 à 50°C durant 5 à 20 heures.
   ▪ pas d'ajout de phytase exogène, la réaction s'opère grâce à l'activité phytasique endogène du son.
   ▪ désactivation enzymatique 90°C pendant 30 minutes.
- tourteau de soja :
   ▪ broyage du tourteau. A titre d'illustration, un broyeur à marteaux équipé d'une grille à 800µm a été utilisé lors des essais Pilote.
   ▪ mise en suspension dans l'eau à raison de 165g de tourteau broyé par kg de suspension.
   ▪ chauffage 40 à 50°C.
   ▪ ajout de phytase exogène (Sumizyme PHY) à la dose de 20g par kg de tourteau brut mis en œuvre, l'activité phytasique endogène étant quasi nulle.
   ▪ durée du traitement 5 à 20 heures, température régulée.
   ▪ désactivation enzymatique 90°C pendant 30 minutes.

A l'issue de ces traitements, les suspensions de son ou de tourteau sont décantées, et/ou clarifiées, et/ou filtrées.

Afin de maximiser la récupération de la matière solubilisée, les boues de décantation, de clarification ou les gâteaux de filtration peuvent être lavés, les eaux de lavages sont alors rassemblées avec le surnageant initial. L'ensemble des solutés peut ensuite être concentré. L'analyse des surnageants et eaux de lavages concentrés récoltés montre que 80% à 90% du phosphore contenu dans le substrat initial est solubilisé.

L'emploi de phytase exogène pour le traitement du son de blé n'améliore pas le rendement de solubilisation/récupération.

La solubilisation du phosphore du son de blé sans recours à une activité phytasique exogène est particulièrement intéressante car l'approvisionnement en phytase non issue d'OGM (obligation du règlement biologique) est difficile.

La solubilisation du phosphore peut ensuite être complétée par les traitements plus classiques d'hydrolyse des protéines du son ou du tourteau (utilisation d'un mélange d'endo et exopeptidases) et de saccharification de l'amidon du son de blé (utilisation d'un mélange amylase et d'amyloglucosidase).

A titre d'exemple, la mise en œuvre de ces différents traitements permet la préparation d'hydrolysats clarifiés dont les compositions sont résumées dans le tableau III.

**Tableau III : Caractéristiques des différents hydrolysats de son de blé ou de tourteau de soja obtenus après traitements successifs afin d'hydrolyser l'acide phytique puis les protéines puis l'amidon.**

| Caractéristique du jus clarifié | | Décoction **son de blé** hydrolyse acide phytique | | | Décoction **tourteau de soja** hydrolyse acide phytique par enzymes exogènes | |
|---|---|---|---|---|---|---|
| | | | + hydrolyse des protéines | | | + hydrolyse des protéines |
| | | | | + hydrolyse de l'amidon | | |
| Matières sèche | g/kg | 51,3 | 81,2 | 81,4 | 83,5 | 116,6 |
| Azote | % MS | 4,9 | 3,8 | 4,0 | 4,2 | 8,3 |
| P₂O₅ | % MS | 13,7 | 6,8 | 7,1 | 3,7 | 2,2 |
| Monomère de sucre | g/kg | 15,6 | 23,8 | 43,2 | | |

Il est particulièrement intéressant de noter que les teneurs en P₂O₅/MS des hydrolysats clarifiés issus du son de blé sont élevées.

### Exemple 3 : Production de levure biologique

Les différents hydrolysats issus du traitement du son de blé ou du tourteau de soja (exemple 2) ont été employés pour la production de levure biologique, selon un procédé industriel classique de production de levures pressées.

Dans l'ensemble, les essais se sont très bien déroulés à tous les niveaux du cycle de fabrication testé.

Les rendements de croissance observés lors des stades levure mères sont comparables à ceux notés habituellement avec autolysat de levure.

Aucune carence en P₂O₅ n'a été notée ni en préfermentation ni en première génération (G1 levure mère) (tableau IV).

Les rendements de croissance sont très bons.

L'activité fermentative de cette série d'essais est globalement très satisfaisante et équivalente à celle des essais de référence. La conservation de la friabilité des levures pressées produites est bonne.

En conclusion, le phosphore issu de l'hydrolyse de l'acide phytique contenu dans le son de blé, ou le tourteau de soja est donc bien assimilable par la levure de boulangerie et permet la fabrication d'une levure de qualité.

**Tableau IV : Taux de phosphore noté lors de la production de levure biologique (le taux de phosphore est exprimée en % P₂O₅/matière sèche levure)**

| | Hydrolysat de son de blé | Hydrolysat de tourteau de soja |
|---|---|---|
| Préfermentation | 5,5 | 4,4 |
| Levure mère (G1) | 1,8 | 1,8 |
| Levure commerciale (G2) | 1,7 à 2,1 | 1,5 à 1,6 |

## Revendications

1. Procédé de culture de levure biologique sur un milieu de culture comprenant des sources de carbone, d'azote et de phosphore d'origines biologiques, **caractérisé en ce que** la source de phosphore est une solution purifiée riche en phosphore, ledit procédé comprenant l'obtention de la solution purifiée riche en phosphore par solubilisation et hydrolyse d'au moins un substrat biologique riche en acide phytique, comprenant de 2 à 18 g de phosphore par Kg de substrat dont 60 à 80% sont sous la forme d'acide phytique, selon le mode suivant :
a. Broyage dudit substrat biologique riche en acide phytique,
b. Mise en suspension du produit obtenu en a,
c. Chauffage de la suspension,
d. Désactivation enzymatique de la suspension,
étant entendu que le terme « biologique » est conforme au règlement (CE) 834/2007 de l'Union Européenne.

2. Procédé selon la revendication 1, **caractérisé en ce que** ledit substrat riche en acide phytique présente aussi une activité phytasique.

3. Procédé selon l'une des revendications 1 ou 2, **caractérisé en ce que** ledit substrat riche en acide phytique est choisi dans le groupe contenant : gluten de maïs, son de riz gras, graines de colza, tourteau de soja, tourteau de tournesol, remoulage demi-blanc de blé tendre, son de blé, seigle, farine de blé tendre.

4. Procédé selon l'une quelconque des revendications 1 à 3, **caractérisé en ce que** le substrat broyé est mis en suspension dans l'eau à raison de 100 à 250 g et de préférence de 150 à 170 g de produit broyé par Kg de suspension.

5. Procédé selon l'une quelconque des revendications 1 à 4, **caractérisé en ce que** la suspension est chauffée à une température comprise entre 40 et 50°C pendant 5 à 20 heures.

6. Procédé selon l'une quelconque des revendications 1 à 5, **caractérisé en ce que** la suspension comprend une phytase.

7. Procédé selon l'une quelconque des revendications 1 à 6, **caractérisé en ce que** le substrat riche en acide phytique est le son de blé.

8. Procédé selon l'une quelconque des revendications 1 à 7, **caractérisé en ce que** le procédé de solubilisation et d'hydrolyse peut être complété par un traitement d'hydrolyse des protéines et de saccharification de l'amidon.

9. Procédé selon l'une quelconque des revendications 1 à 8, **caractérisé en ce que** la source d'azote est préférentiellement du gluten hydrolysé.

10. Procédé selon la revendication 9, **caractérisé en ce que** l'hydrolyse du gluten est réalisée par un cocktail enzymatique comprenant les enzymes suivantes : neutrase, alcalase, cristalase et flavourzyme.

11. Procédé selon l'une quelconque des revendications 1 à 10, **caractérisé en ce que** la source de sucre est la mélasse de canne et/ou de betterave.

12. Procédé selon la revendication 11, **caractérisé en ce que** la source de sucre est préférentiellement constituée d'un mélange de mélasse de canne et de mélasse de betterave dans un rapport compris entre 50/50 et 80/20, et de préférence entre 65/35 et 75/25, et plus préférentiellement proche de 70/30.

13. Procédé selon l'une quelconque des revendications 1 à 12, **caractérisé en ce qu'**il comprend en outre l'ajout de carbonate de sodium, d'acides lactique ou citrique et d'huiles végétales.

## Patentansprüche

1. Verfahren zum Kultivieren von biologischer Hefe auf einem Kulturmedium, das Quellen von Kohlenstoff, Stickstoff und Phosphor biologischen Ursprungs umfasst, **dadurch gekennzeichnet, dass** die Quelle von Phosphor eine phosphorreiche, gereinigte Lösung ist, wobei das Verfahren die Bereitstellung der phosphorreichen, gereinigten Lösung durch Solubilisieren und Hydrolysieren von mindestens einem an Phytinsäure reichen biologischen Substrat, das 2 bis 18 g Phosphor pro kg Substrat enthält, von dem 60 bis 80% in Form von Phytinsäure vorliegen, nach folgendem Verfahren umfaßt:
a. Vermahlen des an Phytinsäure reichen biologischen Substrats,
b. Suspendieren des in a erhaltenen Produktes,
c. Erwärmen der Suspension,
d. Enzymatisches Desaktivieren der Suspension,
wobei der Begriff "biologisch" der Verordnung (EG) Nr. 834/2007 der Europäischen Union entspricht.

2. Verfahren gemäß Anspruch 1, **dadurch gekennzeichnet, dass** das an Phytinsäure reiche Substrat auch eine Phytaseaktivität aufweist.

3. Verfahren gemäß einem der Ansprüche 1 oder 2, **dadurch gekennzeichnet, dass** das an Phytinsäure reiche Substrat aus der Gruppe ausgewählt ist, die: Maisgluten, fette Reiskleie, Rapssamen, Sojaschrot, Sonnenblumenschrot, halbweiße Weichweizengrießkleie, Weizenkleie, Roggen, und Weichweizenmehl enthält.

4. Verfahren gemäß einem der Ansprüche 1 bis 3, **dadurch gekennzeichnet, dass** das gemahlene Substrat in einer Menge von 100 bis 250 g und vorzugsweise von 150 bis 170 g an gemahlenem Produkt pro kg Suspension in Wasser suspendiert wird.

5. Verfahren gemäß einem der Ansprüche 1 bis 4, **dadurch gekennzeichnet, dass** die Suspension 5 bis 20 Stunden auf eine Temperatur zwischen 40 und 50°C erwärmt wird.

6. Verfahren gemäß einem der Ansprüche 1 bis 5, **dadurch gekennzeichnet, dass** die Suspension eine Phytase enthält.

7. Verfahren gemäß einem der Ansprüche 1 bis 6, **dadurch gekennzeichnet, dass** das an Phytinsäure reiche Substrat Weizenkleie ist.

8. Verfahren gemäß einem der Ansprüche 1 bis 7, **dadurch gekennzeichnet, dass** der Solubilisierungs- und Hydrolyseschritt durch eine Behandlung zur Proteinhydrolyse und Stärkeverzuckerung ergänzt werden kann.

9. Verfahren gemäß einem der Ansprüche 1 bis 8, **dadurch gekennzeichnet, dass** die Stickstoffquelle vorzugsweise hydrolysiertes Gluten ist.

10. Verfahren gemäß Anspruch 9, **dadurch gekennzeichnet, dass** die Hydrolyse des Glutens durch einen enzymatischen Cocktail erfolgt, der die folgenden Enzyme umfasst: Neutrase, Alkalase, Cristalase und Flavourzym.

11. Verfahren gemäß einem der Ansprüche 1 bis 10, **dadurch gekennzeichnet, dass** es sich bei der Zuckerquelle um Zuckerrohrmelasse und/oder Rübenmelasse handelt.

12. Verfahren gemäß Anspruch 11, **dadurch gekennzeichnet, dass** die Zuckerquelle vorzugsweise aus einer Mischung von Zuckerrohrmelasse und Rübenmelasse in einem Verhältnis zwischen 50/50 und 80/20, vorzugsweise zwischen 65/35 und 75/25 und stärker bevorzugt nahe an 70/30 zusammengesetzt ist.

13. Verfahren gemäß einem der Ansprüche 1 bis 12, **dadurch gekennzeichnet, dass** es ferner das Zugeben von Natriumcarbonat, Milch- oder Zitronensäure und pflanzlichen Ölen umfasst.

## Claims

1. A method for culturing biological yeast on a culture medium comprising carbon, nitrogen and phosphorus sources of biological origins, **characterized in that** the phosphorus source is a phosphorus-rich purified solution, said method comprising obtaining the phosphorus-rich purified solution by hydrolysis and solubilization of at least one phytic acid-rich biological substrate comprising from 2 to 18 g of phosphorus per kg of substrate, 60% to 80% of which is in the form of phytic acid, according to the following mode:
a. milling said phytic acid-rich biological substrate,
b. suspending the product obtained in a,
c. heating the suspension,
d. enzymatic deactivation of the suspension,
on the understanding that the term "biological" is in accordance with European Union Regulation (EC) 834/2007.

2. The method as claimed in claim 1, **characterized in that** said phytic acid-rich substrate also has a phytase activity.

3. The method as claimed in either of claims 1 and 2, **characterized in that** said phytic acid-rich substrate is chosen from the group comprising: corn gluten, fatty rice bran, rapeseed, soy cake, sunflower cake, half-white common wheat middlings, wheat bran, rye, and common wheat flour.

4. The method as claimed in any one of claims 1 to 3, **characterized in that** the milled substrate is suspended in water in a proportion of from 100 to 250 g and preferably from 150 to 170 g of milled product per kg of suspension.

5. The method as claimed in any one of claims 1 to 4, **characterized in that** the suspension is heated at a temperature of between 40 and 50°C for 5 to 20 hours.

6. The method as claimed in any one of claims 1 to 5, **characterized in that** the suspension comprises a phytase.

7. The method as claimed in one of claims 1 to 6, **characterized in that** the phytic acid-rich substrate is wheat bran.

8. The method as claimed in any one of claims 1 to 7, **characterized in that** the solubilization and hydrolysis method can be added to with a treatment of protein hydrolysis and of starch saccharification.

9. The method as claimed in any one of claims 1 to 8, **characterized in that** the source of nitrogen is preferentially of hydrolyzed gluten.

10. The method as claimed in claim 9, **characterized in that** the gluten hydrolysis is carried out with an enzymatic cocktail comprising the following enzymes: neutrase, alcalase, cristalase and flavourzyme.

11. The method as claimed in any one of claims 1 to 10, **characterized in that** the source of sugar is cane and/or beet molasses.

12. The method as claimed in claim 11 **characterized in that** the source of sugar preferentially consists of a mixture of cane molasses and beet molasses in a ratio of between 50/50 and 80/20 and preferably between 65/35 and 75/25 and more preferentially close to 70/30.

13. The method as claimed in any one of claims 1 to 12, **characterized in that** it also comprises the addition of sodium carbonate, lactic or citric acids and vegetable oils.
